# EUROPEAN PATENT APPLICATION

(11) **EP 2 719 763 A1**
(43) Date of publication of application: **16.04.2014**
(21) Application number: 12187922.5
(22) Date of filing: 10.10.2012
(51) Int. Cl.: C12N 15/82

(54) **Heterodimeric transcription factor involved in PSK synthesis**

(71) Applicant: VIB vzw, 9052 Gent (BE); Universiteit Gent, 9000 Gent (BE)
(72) Inventor: The designation of the inventor has not yet been filed

(57) **Abstract**

The present invention relates to a novel heteromeric plant transcription factor. It relates further to the use of this factor to induce gene transcription, especially genes encoding phytosulfokines, especially PSK2 and PSK5. It relates further to the use of this induction to modulate plant growth and resistance to pathogens.

## Description

The present invention relates to a novel heteromeric plant transcription factor. It relates further to the use of this factor to induce gene transcription, especially genes encoding phytosulfokines, especially PSK2 and PSK5. It relates further to the use of this induction to modulate plant growth and resistance to pathogens.

Plant growth and development depends on continuous generation of new cells in its root and shoot apical meristems. To allow normal root growth, organization and maintenance of the root apical meristem is crucial. The Arabidopsis thaliana root apical meristem is composed of three main zones, determined by their cellular features, namely a stem cell niche (SCN), a proximal meristem zone where cell division occurs, and an elongation/differentiation zone, where cells exit mitosis and start differentiating (Perilli and Sabatini, 2010; Perilli et al., 2012). The SCN, composed of the quiescent center (QC) and its surrounding stem cells, is important for the continuous generation of new cells in the proximal meristem zone and these cells have to maintain their undifferentiated state in order to sustain meristem size (van den Berg et al., 1997; Sarkar et al., 2007). In the QC, WUSCHEL-related homeobox 5 (WOX5) specific expression indirectly controls asymmetric cell divisions in the columella (Sarkar et al., 2007). Presence of the CLE40 signal peptide in the columella restricts expression of WOX5 to the QC cells, thus controlling the rate of columella cell differentiation (Stahl and Simon, 2012). In the SCN, the PIN transporter-dependent auxin maximum results in elevated PLETHORA (PLT) levels, refraining these cells from differentiating (Aida et al., 2004; Dello loio et al., 2008). Going up proximally along the root in the meristematic region, auxin levels decrease, resulting in lowered PLT expression and allowing mitotic cell divisions (Aida et al., 2004). In the SCN, asymmetric cell division required for formation of the endodermis and cortex cell files as well as specification of endodermis is achieved by the expression of the SHORT-ROOT (SHR) and SCARECROW (SCR) transcription factors, members of the GRAS transcription factor family (Helariutta et al., 2000; Sabatini et al., 2003). The root apical meristem size is determined by both the generation of new cells in the meristematic zone, arising from the SCN, and the rate of cell differentiation in the transition zone (Jiang and Feldman, 2005; Moubayidin et al., 2010; Perilli and Sabatini, 2010).

Gibberellins play an important role in determining the meristem size and cortical cell length by promoting the degradation of DELLA growth repressors (Fu and Harberd, 2003; Ubeda-Tomas et al., 2008). This response is transduced by the DELLA antagonist Scarecrow-Like 3 (SCL3), like SHR and SCR a member or the GRAS transcription factor family (Heo et al., 2011; Zhang et al., 2011). Next to gibberellins, peptide hormones are also known to control meristem size. The tyrosine sulfated peptides phytosulfokines PSK, PSY and RGF control root length by maintaining root meristem size and cell expansion in the root elongation-differentiation zone (Kutschmar et al., 2009; Matsuzaki et al., 2010; Whitford et al., 2012). However, neither the signaling components that control the tyrosine sulfated precursor peptide expression, nor their mode of action is well known.

The Ethylene Response Factor 115 (ERF115) is a member or the ERF transcription factors. The ERF family of transcription factors consists of a large family, and has been demonstrated to have important functions in the transcriptional regulation of a variety of biological processes related to growth and development (Nakano et al., 2006). In addition, ERF115 also copurified with several cell cycle proteins, among which the plant specific B-type cyclin dependent kinase CDKB1;2 and three members of the KRP family of cell cycle inhibitors, namely KRP2, KRP4 and KRP5 (Van Leene et al., 2010).

Surprisingly we found that ERF115 forms a functional complex with Scarecrow-Like 21 (SCL21), a member of the GRAS family transcription factors. Even more surprisingly, the ERF115 transcription factor is found to bind directly to the PSK5 promoter in a Tandem Chromatin Affinity Purification (TChAP) assay, and to regulate in complex with SCL21 PSK5 transcription. Correspondingly, the erf115 scl21 double mutant shows a decreased root meristem size and root length, which can be restored upon PSKα supplementation. The ERF115-SCL21 transcription factor complex maintains root meristem size by controlling PSK transcription, which possibly results in CYCD1;1-mediated cell cycle progression.

A first aspect of the invention is an isolated plant transcription factor complex, comprising a protein of the ERF transcription factors, and SCL21 (SEQ ID N°2), or a homologue, orthologue or paralogue thereof. Preferably, said plant transcription factor complex is consisting of a protein of the ERF transcription factors, and SCL21 (SEQ ID N°2), or a homologue, orthologue or paralogue thereof. Even more preferably, said plant transcription factor complex is a heterodimeric complex. In one preferred embodiment, said ERF transcription factor is ERF 115 (SEQ ID N°1), or a homologue, orthologue or paralogue thereof. In another preferred embodiment, said ERF transcription factor is ERF 114, or a homologue, orthologue or paralogue thereof. "Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived. "Orthologues" and "paralogues" encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene. Preferably said ERF115 homologue, orthologue or paralogue comprises an AP2 domain. Even more preferably, said homologue, orthologue or paralogue has at least 50% identities, preferably at least 55% identities, more preferably at least 60% identities with SEQ ID N° 1 (ERF115), as measured by a BLASTp (BLASTP 2.2.27+). As a non-limiting example, said orthologue is encoded by a gene selected from the group consisting of Glyma04g37870, Glyma06g17180, Glyma04g37890 (www.soybase.org), GRMZM2G016434_T01 (www.maizesequence.org). Preferably, said SCL21 homologue, orthologue or paralogue comprises a GRAS domain. Even more preferably, said homologue, orthologue or paralogue has at least 50% identities, preferably at least 55% identities, more preferably at least 60% identities with SEQ ID N° 2(SCL21), as measured by a BLASTp (BLASTP 2.2.27+). As a non-limiting example, said orthologue is encoded by a gene selected from the group consisting of Glyma02g47640, Glyma14g01020 (www.soybase.org) and GRMZM2G116638_T01 (www.maizesequence.org).

Another aspect of the invention is the use of a plant transcription factor according to the invention to induce phytosulphokine genes. Phytosulphokines are known to the person skilled in the art, and include in Arabidopsis PSK1, PSK2, PSK3, PSK4 and PSK5. Preferably the induced phytosulphokines are PSK2 and PSK5, or a homologue, orthologue or paralogue thereof. Even more preferably, said homologue, orthologue or paralogue has at least 50% identities, preferably at least 55% identities, more preferably at least 60% identities with SEQ ID N° 3 or 4 (PSK2; PSK5), as measured by a BLASTp (BLASTP 2.2.27+). As a non-limiting example, said orthologues are selected from a group consisting of Glyma10g40190, Glyma20g27220, Glyma11g04930, Glyma01g40360 and Glyma17g17120 (www.soybase.org).

As it is known that phytosulfokines are important in plant growth regulation and in defense against plant pathogens, another aspect of the invention is the use of a transcription factor complex according to the invention to modulate plant cell and/or plant growth. Preferably, said modulation is an increase in plant growth. The increase may be for the total plant, or for a plant part, such as, but not limited to the leaves, the stem, the roots, the flowers or the seeds. Still another aspect of the invention is the use of a transcription factor complex according to the invention to induce stress resistance in plant. Stress, as used here, may be abiotic or biotic stress. In one preferred embodiment, said stress is abiotic stress, preferably salt stress. In another preferred embodiment, said stress is biotic stress, and the transcription factor complex is used to obtain pathogen and/or pest resistance in plant. A pathogen, as used here, can be any pathogen, including but not limited to fungal pathogens and bacterial pathogens. Preferably, said pathogen is a fungal pathogen. Pests, as used here, may be any plant pest known to the person skilled in the art. Preferably, said pest is a chewing or sucking insect.

Another aspect of the invention is a recombinant plant cell, overexpressing both a gene encoding for an ERF transcription factor and SCL21, or a homologue, orthologue or paralogue thereof. In one preferred embodiment, said ERF transcription factor is ERF 115 (SEQ ID N°1), or a homologue, orthologue or paralogue thereof. In another preferred embodiment, said ERF transcription factor is ERF 114, or a homologue, orthologue or paralogue thereof. Overexpression, as used here means that the expression of the gene is higher than in the non-transformed host plant cell, when cultivated under identical conditions.

Still another aspect of the invention is a transgenic plant, overexpressing both a gene encoding for an ERF transcription factor and SCL21, or a homologue, orthologue or paralogue thereof. In one preferred embodiment, said ERF transcription factor is ERF 115 (SEQ ID N°1), or a homologue, orthologue or paralogue thereof. In another preferred embodiment, said ERF transcription factor is ERF 114, or a homologue, orthologue or paralogue thereof. Overexpression, as used here means that the expression of the gene is higher than in the non-transformed host plant, when grown under identical conditions. The overexpression may be constitutive, or inducible, ensuring that the ERF/SCL21 transcription factor is only overexpressed under certain conditions such as stress or pathogen attack. The overexpression may be systemic or tissue specific. The overexpression can be realized by fusing the gene to a strong promoter, such as but not limited to the CaMV35S promoter. Other strong promoters, for constitutive, inducible, systemic or tissue specific expression are known to the person skilled in the art. Alternatively, the expression is increased by increase of the copy number of the gene.

### BRIEF DESCRIPTION OF THE FIGURES

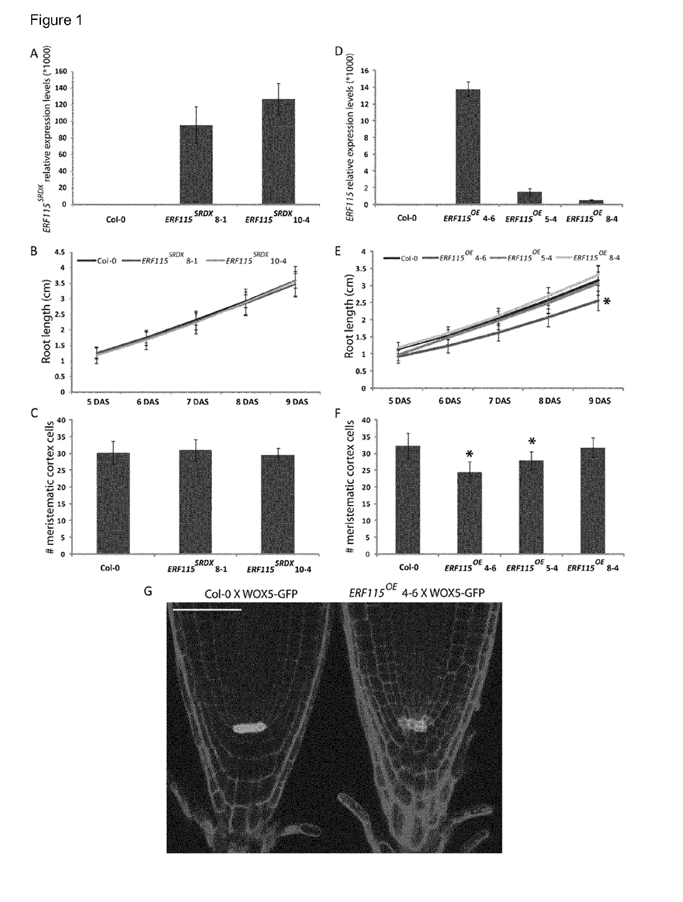

**Figure 1****. ERF115 affects root length and meristem size.**
   (A) Relative ERF115SRDX expression levels in one-week-old old control (Col-0) and ERF115SRDX overexpressing plants. The transcript levels of Col-0 were arbitrarily set to 1. Data represent mean ± SE (n = 3). (B) Root length of lines presented in (A) plotted from 5 to 9 days after sowing (DAS). No effect of ERF115SRDX overexpression on root length could be observed compared to the wild-type (Col-0). Data represent mean ± SD (n > 30). (C) Number of meristematic cortex cells for lines presented in (A). Data represent mean ± SD (n > 14). No effect of ERF115SRDX overexpression on the number of meristematic cortex cells could be observed compared to the wild-type (Col-0). Data represent mean ± SD (n > 14). (D) Relative ERF115 expression levels in one-week-old old control (Col-0) and ERF115OE plants. The transcript levels of Col-0 were arbitrarily set to 1. Data represent mean ± SE (n = 3). (E) Root length of lines presented in (D) plotted from 5 to 9 days after sowing (DAS). Strong ERF115 overexpression results in a decreased root length compared to the wild-type (Col-0). Data represent mean ± SD (n > 28). (F) Number of meristematic cortex cells for lines presented in (D). Data represent mean ± SD (n > 13). No effect of ERF115SRDX overexpression on the number of meristematic cortex cells could be observed compared to the wild-type (Col-0). (G) Confocal images of quiescent center (QC) specific GFP signal, using the WOX5-GFP marker, in one-week-old wild-type and ERF115OE root meristems in combination with propidium iodide stain. ERF115OE results in aberrant divisions and misorganization of the QC. Bars = 50 µm.

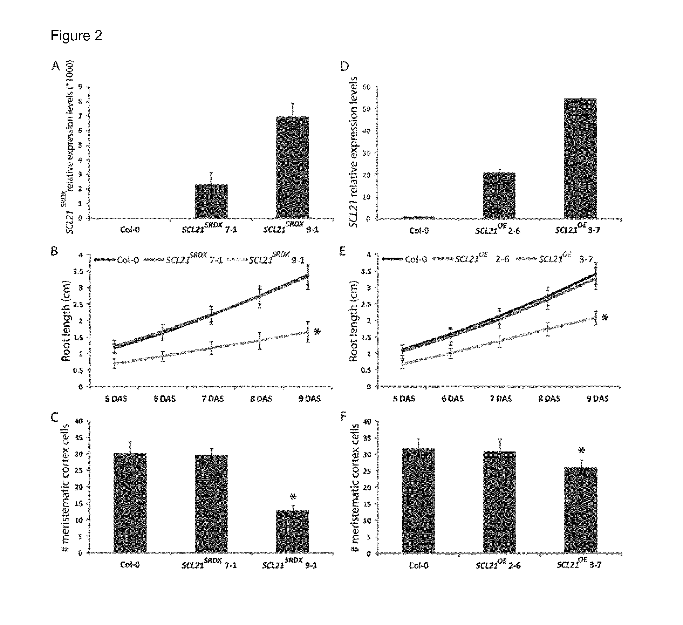

**Figure 2****. SCL21 affects root length and meristem size similar to ERF115.**
   (A) Relative SCL21-SRDX expression levels in one-week-old old control (Col-0) and SCL21SRDX plants. The transcript levels of Col-0 were arbitrarily set to 1. Data represent mean ± SE (n = 3). (B) Root length of lines presented in (A) plotted from 5 to 9 days after sowing (DAS). SCL21-SRDX overexpression results in a decreased root length compared to the wild-type (Col-0). Data represent mean ± SD (n > 31). (C) Number of meristematic cortex cells for lines presented in (A). Data represent mean ± SD (n > 6). SCL21-SRDX overexpression results in a decreased number of meristematic cortex cells compared to the wild-type (Col-0). Data represent mean ± SD (n > 14). (D) Relative SCL21 expression levels in one-week-old old control (Col-0) and SCL21OE plants. The transcript levels of Col-0 were arbitrarily set to 1. Data represent mean ± SE (n = 3). (E) Root length of lines presented in (D) plotted from 5 to 9 days after sowing (DAS). Strong SCL21 overexpression results in a decreased root length compared to the wild-type (Col-0). Data represent mean ± SD (n > 30). (F) Number of meristematic cortex cells for lines presented in (D). Data represent mean ± SD (n > 8). SCL21-SRDX overexpression results in a decreased number of meristematic cortex cells compared to the wild-type (Col-0).

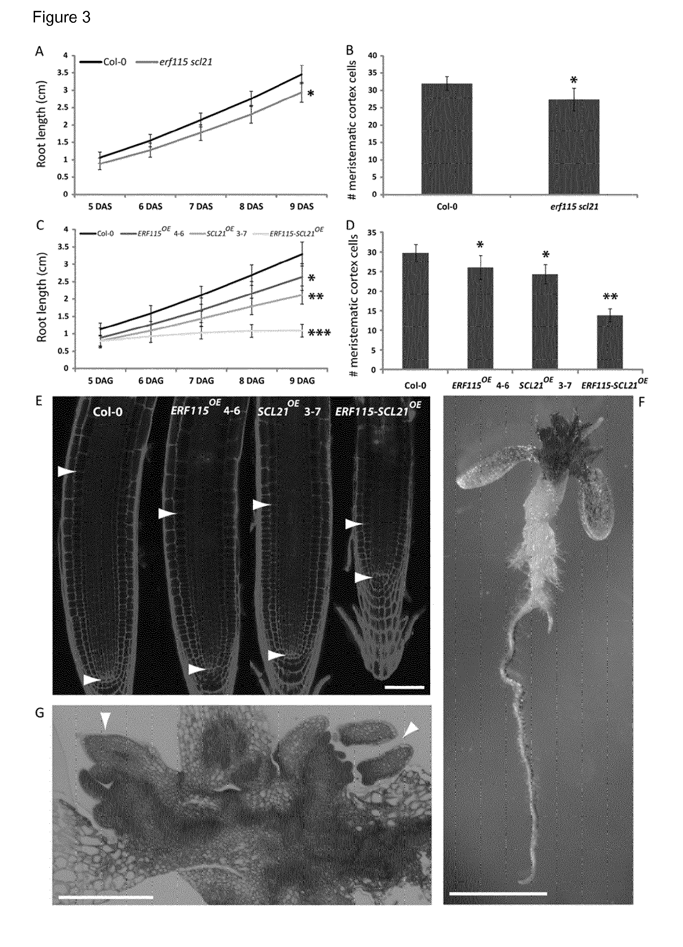

**Figure 3****. ERF115 forms a functional complex with SCL21.**
   (A) Root length of wild-type (Col-0) and the erf115 scl21 double mutant plotted from 5 to 9 days after sowing (DAS). The erf115 scl21 double mutant has a significant decreased root length compared to wild-type (*p-value < 0.05, Student's t-test). Data represent mean ± SD (n > 32). (B) Number of meristematic cortex cells for lines presented in (A). The erf115 scl21 double mutant has a significant reduced meristematic cortical cell number compared to wild-type (*p-value < 0.05, Student's t-test). Data represent mean ± SD (n > 14). (C) Root length of wild-type (Col-0) ERF115OE 4-6, SCL210E 3-7 and ERF115-SCL21OE mutants plotted from 5 to 9 days after sowing (DAS). The ERF115OE mutant has a significant decreased root length compared to wild-type (*p-value < 0.05, Student's t-test). The SCL21OE mutant has a significant decreased root length compared to the ERF115OE mutant and the wild-type (**p-value < 0.05, Student's t-test). The ERF115-SCL21OE mutant has significant decreased root length compared to the single overexpression mutants and the wild-type (***p-value < 0.05, Student's t-test). Data represent mean ± SD (n > 8). (D) Number of meristematic cortex cells for lines presented in (C). The ERF115OE and SCL21OE single mutants have a similar decreased meristematic cortical cell number compared to wild-type (*p-value < 0.05, Student's t-test). The ERF115-SCL21OE mutant has significant decreased meristematic cortical cell number compared to the single overexpression mutants (**p-value < 0.05, Student's t-test). Data represent mean ± SD (n > 7). (E) Representative confocal microscopy images of one-week-old root meristems stained with propidium iodide of lines presents in (C). Arrowheads indicate the meristem size based on the cortical cell length. Bars = 50 µm. (F) Image of 12-days-old ERF115-SCL21OE seedling. Bars = 0.5 cm. (G) Section of 12-days-old ERF115-SCL210E mutant shoot. Arrowheads indicate multiple apical meristems in the shoot. Bars = 0.1 mm.
**Figure 4****. Tandem affinity purification based interactome.**
   Interactions identified from TAP experiments using ERF114, ERF115 and SCL21 as bait. Arrowheads point to the interacting prey; double arrowheads indicate both interact as prey.
**Figure 5****. ERF114 overexpression affects root length.**
   (A) Relative ERF114 expression levels in one-week-old old control (Col-0) and three independent ERF114OE lines. The transcript levels of Col-0 were arbitrarily set to 1. Data represent mean ± SE (n = 3). (B) Root length of lines presented in (A) plotted from 5 to 9 days after sowing (DAS). Strong ERF114 overexpression results in a decreased root length compared to wild-type (*p-value < 0.05, Student's t-test). Data represent mean ± SD (n > 24). (C) Number of meristematic cortex cells for lines presented in (A). Data represent mean ± SD (n > 7). Strong ERF114 overexpression lines show a decrease in the number of meristematic cortex cells compared to the wild-type (Col-0) (*p-value < 0.05, Student's t-test). (D) Representative confocal microscopy images of one-week-old root meristems stained with propidium iodide of wild-type and the ERF114OE 8-5 mutant. Arrowheads indicate the meristem size based on the cortical cell length. Bars = 50 µm.

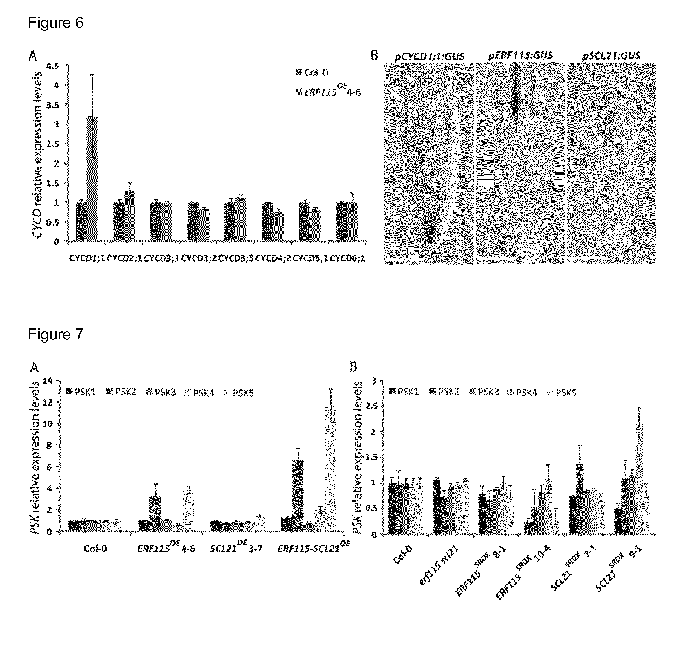

**Figure 6****. ERF115 has an effect of CYCD1;1 expression.**
   (A) Relative expression levels of several D-type cyclins in 5 DAS old control (Col-0) and ERF115OE root tips. The transcript levels of Col-0 were arbitrarily set to 1. Data represent mean ± SE (n = 3). (B) Microscopy image of GUS stained 4 DAS old root tips of promoter CYCD1;1:-, ERF115:- and SCL21 :GUS lines. Bars = 0.1 mm.
**Figure 7****. ERF115 and SCL21 regulate PSK transcription.**
   (A) Relative expression levels of PSKs in 5 DAS old control (Col-0), ERF115OE, SCL210E and ERF115-SCL21OE mutant roots. (B) Relative expression levels of PSKs in 5 DAS old control (Col-0), erf115 scl21 double mutant, ERF115SRDX and SCL21SRDX mutant roots. The transcript levels of Col-0 were arbitrarily set to 1. Data represent mean ± SE (n = 3).

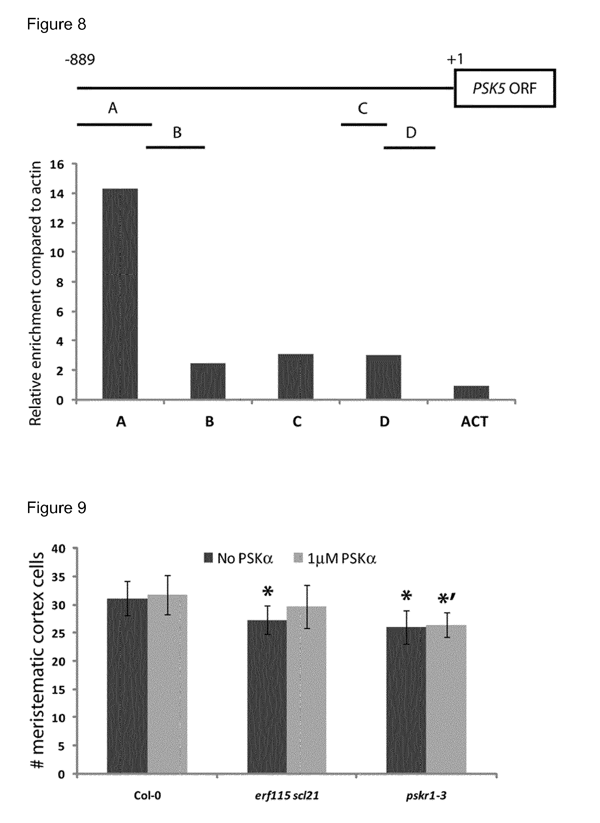

**Figure 8****. ERF115 binds the PSK5 promoter.**
   Promoter element enrichments after tandem chromatin affinity purification from TAP-tagged ERF115 expressing Arabidopsis cell suspension cultures with actin as a negative control (n = 1).
**Figure 9****. erf115 scl21 mutant root growth restoration by PSKα.**
   Number of meristematic cortex cells from one-week-old root tips of wild-type (Col-0), erf115 scl21 double and pskr1-3 mutants. Both erf115 scl21 double and pskr1-3 mutants have a significantly decreased number of meristematic cells compared to the wild-type under control conditions (*p-value < 0.05, Student's t-test). Upon PSKα treatment, only the pskr1-3 mutant has a decreased number of meristematic cells compared to the wild-type (*'p-value < 0.05, Student's t-test). Data represent mean ± SD (n > 13).

### EXAMPLES

### Materials and methods to the examples

### Mutant lines

The erf115 (SALK_021981) and scl21 (SALK_146085) mutants were obtained from the SALK T-DNA insertion collection. The ERF115OE and SCL21OE lines were generated through recombination of the respective genomic ORFs into the pK7GW2 and pH7GH2 expression vectors, respectively, under control of the CaMV 35S promoter via gateway recombination. For the SRDX dominant negative lines, the ERF115 and SCL21 cDNA ORFs were fused in front of the SRDX sequence via PCR adaptor ligation and recombined into the pK7GW2 and pH7GH2 expression vectors, respectively, under control of the CaMV 35S promoter via gateway recombination. For GUS reporter constructs, the 2000 bp and 664 bp promoter regions upstream of the ERF115 and SCL21 start codons, respectively, were recombined into the pMK7S*NFm14GW plasmid, in front of the β-glucuronidase ORF.

### Plant medium and growth conditions

Plants were grown under a long-day/short-night regime (16 h light/8 h darkness) at 21°C on agar-solidified culture medium (Murashige and Skoog [MS] medium, 10 g/l saccharose, 0.43 g/l 2-(N-morpholino)ethanesulfonic acid [MES], and 0.8% plant tissue culture agar). ERF115OE lines, ERF115SRDX and SCL21 SRDX transgenic lines were screened using 45 mg/l kanamycin selection; SCL21OE plants were selected using 15 mg/l hygromycin. For the PSKα treatments, plants were grown for the indicated time on MS medium supplemented with 1 µM PSKα peptide from PolyPeptide group (#SC1465). For the root length measurements, plants were grown for the indicated time and analyzed using the ImageJ 1.41 software. For leaf size measurement, 3-week-old leaves were harvested, cleared with ethanol and fixed using lactic acid. The leaf size was analyzed using the ImageJ 1.41 software. For callus formation induction, one week old etiolated hypocotyl segments were cultured for three weeks on callus inducing medium (1 x MS salts, 10 g/l saccharose, 0.5 g/l 2-(N-morpholino)ethanesulfonic acid [MES], 100 mg/l myo-inositol, 0.3% Phytagel, 30 µg/l 2,4-dichlorophenoxyacetic acid [2,4-D], and 1 mg/l kinetin). For MG132 treatments, plants were grown on vertical MS plates for six days and transferred over night to liquid MS medium containing 100 µM MG132 from BostonBiochem (#I-130) or DMSO as a control.

### Confocal and scanning electron microscopy

Root meristems were analyzed by the Zeiss Axiovert 100M confocal laser scanning microscopy. Plant material was incubated for 2 min in a 10 µM propidium iodide (PI) solution to stain the cell walls and observed under epifluorescent light with the appropriate filter set (excitation filter, 535-550 nm; dichroic mirror, 565 nm; barrier filter, 590 nm). GFP fluorescence was detected with a 500- to 550-nm band-pass emission filter. Scanning electron images were acquired using the TM-1 000 Tabletop electron microscope (Hitachi).

### RT-qPCR analysis and microarray analysis

RNA was isolated from the respective tissues using the RNeasy isolation kit from Qiagen. DNase treatment was performed using the RQ1 RNase-Free DNase (Promega) in prior to cDNA synthesis using the iScript cDNA Synthesis Kit (Bio-Rad). Relative expression levels were determined by RT-qPCR using the LightCycler 480 Real-Time SYBR green PCR System (Roche). Two different reference genes were used for normalization, depending on the source of the tissue. ACT and CAK2 were used for whole plant material, PAC1 and EMB2386 for roots.

In three independent experiments, total RNA was isolated from root meristem material of wild type Col-0, ERF115OE and erf115 mutant root tips with RNeasy Plant mini kit (Qiagen). Plants were grown on nylon meshes (Prosep), placed on vertical plates containing MS. Root tips were harvested 5 days after sowing using a scalpel. Each of the different RNA samples of wild-type and transgenic plants were hybridized to 9 Genechip® Arabidopsis ATH1 Genome Arrays (Affymetrix, Santa Clara, CA). Reverse transcription, labeling, hybridization and scanning were performed according to the manufacturer's instructions (Affymetrix; https://www.affymetrix.com/) at the Nucleomics Core Facility (Leuven, Belgium; http://nucleomics.be). Raw data (CEL-files) were processed with the statistical algorithm Robust Multiarray Average (RMA) (Irizarry et al., 2003) using the affy package of R/Bioconductor (Gautier et al., 2004) and subjected to an analysis of variance (ANOVA) using the TIGR MultiExperiment Viewer (MeV) of TM4 (Saeed et al., 2003). A multiple testing correction was performed on the P-values of the F-statistics to adjust the false discovery rate using QVALUE (http://genomine.org/qvalue/) (Storey and Tibshirani, 2003) with λ ranging from 0.0 to 0.95 by 0.05. Genes with P-values < 0.001 and Q-values ≤ 0.042 were retained for further analysis. Within the significant profiles, WT and transgenic profiles were also compared pair-wise, selecting genes with a P-value < 0.002 and Q-value < 0.05. Significant profiles were further processed: Expression values were obtained by averaging the inverse log2-transformed normalized values of signal intensities of the three replicates. Fold changes were obtained using the averaged expression values of the transgenics relative to the wild type samples.

### Tandem affinity purifications and tandem chromatin affinity purification

Generation of TAP-tagged constructs, Arabidopsis cell suspension culture transformation and cultivation (Van Leene et al., 2007). Tandem affinity purification of protein complexes was done using the GS tag (Burckstummer et al., 2006) followed by protein precipitation and separation (Van Leene et al., 2008). For the protocols of proteolysis and peptide isolation, acquisition of mass spectra by a 4800 MALDI TOF/TOF Proteomics Analyzer (AB SCIEX), and MS-based protein homology identification based on the TAIR genomic database (Van Leene et al., 2010).

For tandem chromatin affinity purification construct generation, the HBH tag sequence was amplified by PCR using the pFA6HBH-KanMX6 plasmid as template (Tagwerker et al., 2006) and cloned into the pDONRP2RP3 ENTRY vector by BP recombination reaction according to the manufacturer's instructions (Invitrogen). The P35S:ERF115 HBH vector was obtained by Multisite Gateway LR reaction between pEntryL4R1 P35S, pEntryL1 L2 ERF115, and pEntryR2L3 HBH and the destination vector pK7GW43D (Van Leene et al., 2007). For tandem chromatin affinity purification, two-day-old exponentially growing cell cultures were treated with 0.75% formaldehyde for 10 min. Crosslinking was stopped by addition of 0.25 M glycine during 10 min. Cells were filtered and washed with PBS pH 7.2 (0.14 M NaCl, 2.7 mM KCI, 10 mM PO43-). The harvested material was ground in liquid nitrogen and solubilized in NLB buffer (50 mM HEPES pH 7.5, 150 mM NaCl, 0.5 mM EDTA, 1% Triton X-100, 0.1% sodium deoxycholate, 0.1% SDS, 1 µg/ml pepstatin A, 1 µg/ml aprotinin, 1 µg/ml leupeptin, 1 mM PMSF) with an Ultra-Turrax T25 mixer. Chromatin was fragmented on ice with a probe sonicator to obtain ~200-800-bp fragments. After sonication, the suspension was centrifuged twice for 20 minutes at 16000 rpm, the protein concentration was determined by using the Bio-Rad protein assay kit, and the extract was used for chromatin precipitation.

Extract containing 200 mg protein was adjusted to 10 mM imidazole and incubated overnight on a rotating wheel with 300 µl Ni-NTA Superflow (Qiagen). The NiNTA resin was washed three times with 10 ml NLB + 10 mM imidazole and complexes were eluted with two times 1.5 ml NLB + 150 mM imidazole. Next, the eluates from three NiNTA purifications were pooled on 100 µl Streptavidin Sepharose. After 3-h incubation on a rotating wheel, the Streptavidin Sepharose beads were washed with 10 ml NLB + 10 mM imidazol and three times with 1ml NLB containing 750 mM NaCl and increasing SDS concentrations (0.1%, 0.5% and 1 %).

Finally beads were washed with 15 ml TE buffer (10 mM TrisCl pH8, 500 mM NaCl, 1 mM EDTA) and the bound E2Fa-DNA complexes eluted and reverse crosslinked by incubation overnight at 65°C on a rotating wheel in 1 ml 10 mM TrisCl pH8, 1 mM EDTA, 0.5 M NaCl, 1% SDS + 0.5 µl RNAse A (100 mg/ml). Next, the eluates were incubated with 100 µg Proteinase K for 2 h at 42°C on a rotating wheel and the DNA extracted by phenol/chloroform/IAA followed by purification using Qiaquick PCR purification kit and DNA quantification by the Quant-iT dsDNA High Sensitivity kit (Invitrogen).

Scanning of the PSK5 promoter for enriched elements was performed using qPCR. Primer combinations were designed using the NCBI primer-BLAST webtool (http://www.ncbi.nlm.nih.gov/tools/primer-blast). Calculation of the relative enrichment of promoter fragments was performed as described in Chapter 1.

### GUS staining assay and sections

Samples were grown for the indicated time and fixed in ice cold 80% acetone. After three washes using phosphate buffer (14 mM NaH2PO4; 36 mM Na2HPO4), samples were incubated in assay buffer (0.5 mg/ml X-Gluc; 0.165 mg/ml potassium ferricyanide(III); 0.211 mg/ml potassium hexacyanoferrate(II) trihydrate dissolved in phosphate buffer) at 37°C until sufficient staining was observed. Samples were cleared using 70% ethanol in prior to fixing and mounting on slides using lactic acid. Samples were analyzed using the Olympus BX51 microscope.

For sections, plant material was fixed for several days at 4°C using fixing solution (1% gluteraldehyde, 4% formaldehyde) in prior to dehydratation by washing using increasing ethanol concentrations (30%, 50%, 70%, 85%, 95%) for 2 hours each. Infiltration was performed using increasing concentrations of technovit (33%, 50%, 66% technovit in ethanol) for 2 hours each. Final imbedding was performed using 100% technovit in prior to sectioning using a microtome. Cell walls were stained using 0.05% ruthenium red solution. Samples were analyzed using the Olympus BX51 microscope.

### Accession numbers

Sequence data from this article can be found in the Arabidopsis Genome Initiative or GenBank/EMBL databases under the following accession numbers: CCS52A1 (At4G29910); CCS52A2 (At4G11920); CYCD1;1 (At1G70210); ERF114 (At5G61890); ERF115 (At5G07310); PSK1 (At1G13590); PSK2 (At2G22860); PSK3 (At3G44735); PSK4 (At3G49780); PSK5 (At5G65870); PSKR1 (At2G02220); PSY (At5G58650); RGF1 (At5G60810); RGF2 (At1G13620); SCL21 (At2G04890).

### Example 1: ERF115 affects root growth

Because ERF115 was found to interact with several cell cycle proteins, it was tested whether ERF115 could play a role during development. To this end, we analyzed an ERF115 T-DNA insertion mutant, which was found to show about 10% residual transcript. Using this mutant, the root growth was analyzed from five until nine days after sowing, however, no clear effects could be observed compared to control plants (data not shown). Because ERF115 belongs to the large family of ERF transcription factors, we postulated that other ERF family members might work redundantly with ERF115. Therefore, a dominant negative transcription factor was generated by fusing ERF115 to the SRDX dominant repressor (Figure 1A). In this way, possible redundancy from closely related ERFs should be avoided. However, when the root length of the dominant negative ERF115SRDX plants was investigated, again no reduction in root length was observed (Figure 1 B). Correspondingly, no significant reduction in the root meristem size of ERF115SRDX plants could be observed compared to wild type plants (Figure 1 C).

Because ERF115 loss-of-function and dominant negative mutants did not show obvious root phenotypes, we investigated whether overexpression of ERF115 would have an effect on root growth. Transgenic plants expressing the genomic sequence of ERF115 under control of the constitutive CaMV 35S promoter, were generated (Figure 1 D). These transgenics appeared smaller than wild type plants. Plants strongly overexpressing ERF115 showed a clear reduction in root length compared to wild-type roots (Figure 1 E). To determine the cause of the reduction in root length, the meristem size, represented by the number of meristematic cortex cells, was examined through confocal microscopy. A clear reduction in the number of meristematic cortex cells was observed for the ERF115OE plants, compared to wild type plants (Figure 1 F). Additionally, we noticed aberrant divisions in the QC of ERF115OE root meristems, similar to those described for ccs52a2 knockout plants (Vanstraelen et al., 2009). To investigate this phenotype in more detail, we introduced the WOX5-GFP marker line, which is exclusively expressed in the QC cells, and the F1 generation was investigated using confocal microscopy. This analysis confirmed the occurrence of aberrant cell divisions and misorganization in the QC of ERF115OE plants in the three independent ERF115OE lines, being most outspoken in the strongest ERF115OE line (Figure 1 G).

### Example 2: ERF115 forms a functional complex with SCL21

Because transcription factors most often operate as homo- or hetero-dimer complexes, a TAP purification was performed using ERF115 as bait. Only two proteins copurified reproducibly with ERF115, namely dihydrodipicolinate reductase family protein and Scarecrow-Like 21, a member of the GRAS family of transcription factors (Table 1). To investigate whether ERF115 and SCL21 indeed function as a complex, we investigated the effect of SCL21 misexpression on plant growth. Therefore, a SALK T-DNA insertion mutant harboring an insertion in the SCL21 promoter region was isolated, showing only 12% residual transcript. Plants lacking a fully functional SCL21 gene displayed a significantly increased root length compared to wild type plants, albeit small. Correspondingly, when observing the meristem size of scl21 mutants, a small but significant increase in meristematic cortex cell number was observed compared to wild type plants. However, because SCL21, like ERF115, is part of a large family, the possibility of functional redundancy again could not be excluded. Thus, like for ERF115, transgenic plants expressing the dominant negative SCL21-SRDX fusion under control of the CaMV 35S promoter were generated (Figure 2A) and the root length was determined. A severe reduction in root length was observed for plants overexpressing the SCL21-SRDX fusion construct compared to wild type plants (Figure 2B). When observing the root meristem, a strong reduction in the number of meristematic cortex cells could be observed in SCL21 SRDX roots compared to the wild type, which corresponds to the reduced root length (Figure 2C).

**Table 1. Tandem affinity purified proteins using ERF115 as bait.**

| **AGI** | **Protein Name** | **Protein MW** | **Peptide Count (a)** | **Protein Score (b)** | **Best Ion Score (c)** |
|---|---|---|---|---|---|
| At2g44040 | dihydrodipicolinate reductase protein | 37754 | 10 | 786 | 162 |
| At5g07310 | ERF115 | 29388 | 8 | 613 | 174 |
| At2g04890 | SCL21 | 46951 | 7 | 97 | 39 |

| | | | | | |
|---|---|---|---|---|---|
| a. The number of peptides with unique sequences matching the selected protein. b. Mascot score derived from the peptide mass fingerprint, combined with tandem mass spectrometry data. c. Ion scores are mascot scores from individual peptide sequence data. The bait is depicted in italic. | | | | | |

In addition to SCL21 loss-of-function mutants, gain-of-function plants were generated by expressing SCL21 under control of the CaMV 35S promoter (Figure 2D). Similar to the SCL21 SRDX lines, strong SCL21OE seedlings showed a strong decrease in root length compared to wild type, whereas weak SCL21OE plants showed no significant root length reduction (Figure 2E). This reduction in root length appeared to be due to a reduction in meristematic cortex cells in the root meristem (Figure 2F), comparable to ERF115OE lines. However, in contrast to ERF115 overexpression, overexpression of SCL21 did not appear to have any effect on QC organization.

To test whether ERF115 and SCL21 could work together as a complex, an erf115 scl21 double mutant was generated and its root length was studied. A significant reduction in root length was observed for the erf115 scl21 double mutant, compared to the single mutants and wild type plants (Figure 3A). When determining the meristem size of the erf115 scl21 double mutant, a significant reduction in the number of meristematic cortex cells was observed, compared to the wild type (Figure 3B). Also, the effect of ERF115-SCL21 co-overexpression on growth was analyzed. Plants overexpressing both ERF115 and SCL21 were generated via crossing and the F1 was analyzed. Double overexpression seedlings displayed a root growth arrest at 5 days after germination (Figure 3C). The root meristem size was severely reduced in size compared to the single ERF115OE and SCL210E mutants, the QC was misorganized and additional layers in the columella could be observed (Figure 3D and 3E).

Contrary to the root, the shoot part appeared to be overproliferating with the shoot meristem showing many leaf like structures (Figure 3F) due to the appearance of several shoot apical meristems (Figure 3G), whereas the hypocotyl enlarged to a callus structure (Figure 3F). Two weeks after germination, growth was completely arrested, except for additional swelling of the hypocotyl. These observations indicate that co-overexpression of ERF115 and SCL21 has a dramatic effect on plant development.

### Example 3: ERF114 can substitute ERF115 to form a redundant complex with SCL21

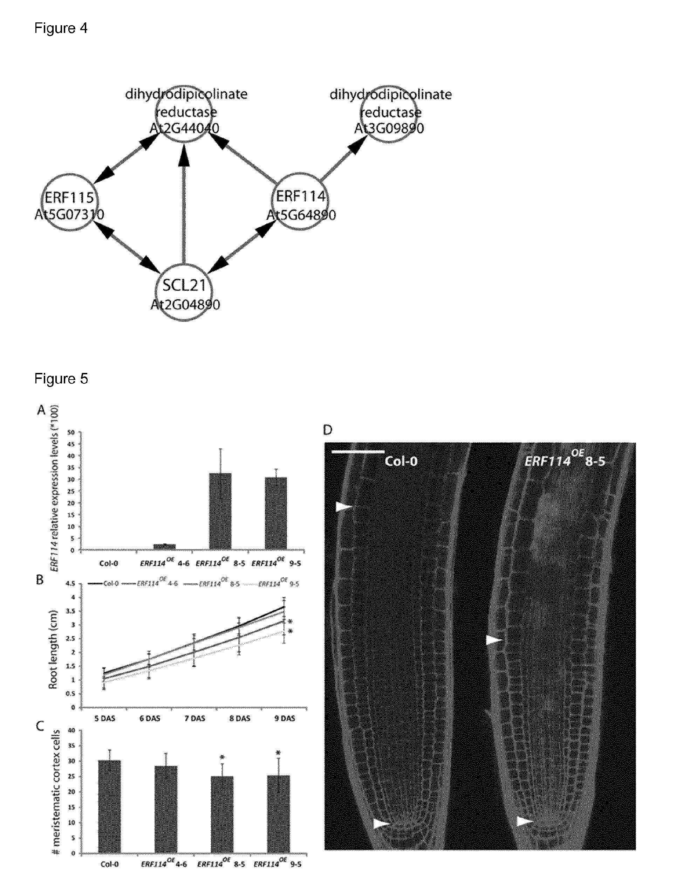

Because both ERF115 and SCL21 belong to large gene families and single mutation of either results in only little or no plant growth effects, we wondered whether there might be redundant factors involved in the process controlled by the ERF115-SCL21 transcription factor complex. To investigate this, a TAP purification was performed using SCL21 as bait. Again, ERF115 and the dihydrodipicolinate reductase family protein copurified with SCL21, confirming the previous observed interaction. However, a new interactor copurified with SCL21 as well, namely ERF114, belonging to the same subfamily of ERF transcription factors as ERF115 (Figure 4) (Nakano et al., 2006). Reverse TAP using ERF114 as bait confirmed the interaction with SCL21 (Figure 4).

On the protein level, ERF114 and ERF115 share a 64% degree of homology, thus it was hypothesized that ERF115 and ERF114 might operate redundantly. No T-DNA insertion line for ERF114 was available, thus plants overexpressing ERF114 under control of the CaMV 35S promoter were generated (Figure 5A) and root length was determined. Similar to ERF115OE plants, overexpression of ERF114 resulted in a decrease in root length compared to wild type plants (Figure 5B). When determining the root meristem size of ERF114OE plants, a significant reduction in the number of meristematic cortex cells compared to wild type plants was observed, again similar as for ERF115OE plants (Figure 5C and 5D). However, in contrast to ERF115OE, ERF114OE QC cells did not show any aberrant divisions or misorganization (Figure 5D). These data suggest ERF114 and ERF115 can perform similar effects on root growth, because overexpression of ERF114 and ERF115 has a similar effect on root length and meristem size.

### Example 4: ERF115 affects expression of CYCD1;1

Seeing how misexpression of ERF115 and SCL21 causes strong defects in growth, we wondered what the targets of this transcription factor complex could be. The SCR transcription factor, a member of the GRAS transcription factors, has been shown to bind the CYCD6;1 promoter to regulate its transcription in Arabidopsis thaliana cortex-endodermis initial cells (Sozzani et al., 2010). Thus, it was tested whether the ERF115-SCL21 transcription factor complex might regulate the expression of D-type cyclins in Arabidopsis root meristems. To verify this, root tips of 5 DAS old ERF115OE and wild type plants were harvested and expression levels of several D-type cyclins were assessed via RT-qPCR. Out of all tested cyclins, only CYCD1;1 showed a 3.2-fold upregulation upon ERF115 overexpression (Figure 6A). The expression of CYCD1;1 did not appear to be downregulated in erf115 mutant root tips, but this could be due to redundancy between ERF114 and ERF115.

Next, we aimed to confirm whether ERF115-SCL21 regulates CYCD1;1 transcription, through investigating whether ERF115 and SCL21 are co-expressed in tissues where CYCD1;1 expression occurs. Therefore, a GUS staining was performed on 4 DAS old plants harboring the β-glucuronidase gene under control of the CYCD1;1, ERF115 or SCL21 promoter. GUS staining of pERF115:GUS and pSCL21:GUS harboring plants showed that ERF115 and SCL21 are expressed in the vascular tissue. Sections indicate that ERF115 is predominantly expressed in the phloem. In contrast, CYCD1;1 expression appeared to be restricted to the QC and columella cells, and completely absent in tissues where expression of ERF115 and SCL21 is present (Figure 6B). These findings suggested that ERF115-SCL21 might affect CYCD1;1 expression in an indirect manner.

### Example 5: ERF115-SCL21 regulates PSK transcription

To identify potential direct targets of the ERF115-SCL21 complex, ERF115 mutant root tips were subjected to microarray analysis via the ATH1 platform. Analysis revealed that one of the strongest upregulated genes in ERF115OE plants was the phytosulfokine 5 precursor (PSK5) gene, displaying an 8.1-fold induction compared to control root tips (Table 2). The Arabidopsis thaliana genome encodes 5 phytosulfokine precursor genes, which give rise to a sulfonated pentapeptide signaling molecule, shown to be involved in root growth in Arabidopsis and callus formation in carrot hypocotyls (Yang et al., 2001; Matsubayashi et al., 2004; Kutschmar et al., 2009). Next to PSK5, the other PSK precursor genes were differentially expressed in ERF115OE root tips. RT-qPCR analysis confirmed the observed PSK5 upregulation. In addition, PSK2 also appeared to be upregulated in ERF115OE root tips, whereas PSK1 and PSK3 expression did not appear to be affected. Only PSK4 showed a 36% downregulation in ERF115OE root tips (Figure 7A). Next, we verified the PSK expression levels in SCL210E roots. In contrast to ERF115, strong overexpression of SCL21 only resulted in a minor upregulation of PSK5 in roots, whereas PSK1 to PSK4 did not show any significant difference compared to wild type expression levels (Figure 7A). Next, we wondered whether the PSK expression would be additively upregulated in ERF115-SCL21 co-overexpressing roots. Expression of PSK1 and PSK3 did not alter noticeably upon co-overexpression compared to the single ERF115OE and SCL21OE mutants. In contrast, expression of PSK2, PSK4 and especially PSK5 was strongly enhanced in the ERF115-SCL21OE roots compared to the single ERF115OE and SCL21 OE lines (Figure 7A).

Next, PSK transcription levels were analyzed in the erf115 scl21 double mutant, and the ERF115SRDX and SCL21SRDX dominant negative roots. PSK2 showed a 28% downregulation in erf115 scl21 double mutant roots compared to wild type roots, whereas the other PSKs did not show a significant reduction in transcripts (Figure 7B). PSK1, PSK3 and PSK5 transcripts were found to be 50% reduced in ERF115SRDX dominant negative lines, whereas PSK2 and PSK4 show no obvious downregulation (Figure 7B). In SCL21SRDX dominant negative seedlings, mainly the PSK1 and PSK5 transcription levels showed downregulation between 20 and 40% (Figure 7B). Combined, these data suggest that ERF115 and SCL21 control expression of PSK, as observed from a PSK upregulation in ERF115OE and SCL21OE plants and downregulation in the ERF115SRDX and SCL21SRDX dominant negative mutants.

**Table 2. Top 20 most upregulated genes in ERF115OE compared to wild type root tips found by microarray analysis.**

| **AGI** | **Protein name** | **Fold induction** |
|---|---|---|
| At2G37640 | EXP3 | 10,696 |
| At4G36430 | peroxidase, putative | 8,712 |
| At5G65870 | ATPSK5 (PHYTOSULFOKINE 5 PRECURSOR) | 8,144 |
| At3G59930 | Encodes a defensin-like (DEFL) family protein | 6,948 |
| At5G39580 | peroxidase, putative | 6,625 |
| At4G38860 | auxin-responsive protein, putative | 6,267 |
| At2G05520 | GRP-3 (GLYCINE-RICH PROTEIN 3) | 6,001 |
| At2G39690 | unknown protein | 5,229 |
| At3G13980 | unknown protein | 4,874 |
| At5G48070 | XTH20 (xyloglucan endotransglucosylase/hydrolase) | 4,736 |
| At3G56980 | BHLH039 (DNA binding / transcription factor) | 4,445 |
| At1 G77380 | AAP3 (amino acid transmembrane transporter) | 4,279 |
| At1 G72450 | JAZ6 | 4,059 |
| At1 G34540 | CYP94D1 (electron carrier) | 3,563 |
| At2G34180 | CIPK13 (CBL-INTERACTING PROTEIN KINASE 13) | 3,336 |
| At1 G69530 | ATEXPA1 (ARABIDOPSIS THALIANA | 3,241 |
| | EXPANSIN A1) | |
| At5G67400 | peroxidase 73 (PER73) (PRXR11) | 3,105 |
| At1G47400 | unknown protein | 3,094 |
| At1G30700 | FAD-binding domain-containing protein | 3,067 |
| At4G18510 | CLE2 (CLAVATA3/ESR-RELATED) | 3,005 |

### Example 6: ERF115 binds the PSK5 promoter

The strong response of PSK transcription upon ERF115 and SCL21 misexpression suggests that the ERF115-SCL21 transcription factor complex directly controls PSK transcription. To test this hypothesis, tandem chromatin affinity purification was performed using TAP-tagged ERF115 as bait. Tandem chromatin affinity purification is similar to the standard chromatin immuno-precipitation, but a double affinity purifying step using the TAP-tag is performed instead of a single pull down. A region of 900 bp upstream of the PSK5 start codon was scanned using qPCR and enrichment was observed of the promoter region between 800 and 900 bp upstream (Figure 8). Sequencing data confirmed the enrichment of this PSK5 promoter region (data not shown). This indicates that ERF115 indeed binds the PSK5 promoter in order to regulate its expression. Moreover, the previously reported expression profile of PSK5 in the root meristem corresponds with that of ERF115 and SCL21 (see Figure 6B and Kutschmar et al., 2009).

### Example 7: PSKα supplementation restores the erf115 scl21 double mutant root meristem phenotype

Because PSKα has been shown to play a role in root growth and determination of root meristem size, we hypothesized that the reduced meristem size of the erf115 scl21 mutant could be due to the decreased PSK levels. Thus, addition of exogenous PSKα peptide should be able to restore the root meristem size of erf115 scl21 double mutants. To test this, plants were grown on MS medium supplemented with 1 µM PSKα peptide for one week. As a control, plants lacking the PSK receptor 1 (PSKR1) were used. When grown under control conditions, again a significant reduction in meristematic cortex cells for the erf115 scl21 double mutant could be observed, compared to wild type plants. The pskr1-3 mutant also displayed a significant reduced number of meristematic cortex cells (Figure 9). When the number of meristematic cortex cells was determined for plants grown on 1 µM PSKα, no effect could be seen for wild type plants. Contrary, application of the peptide to the erf115 scl21 double mutant restored the amount of cortical cells to the wild-type number. In contrast, the root meristem size of the pskr1-3 mutant was not restored upon PSKα addition (Figure 9). These data indicate that the reduction in meristem size of erf15 scl21 mutants is due to insufficient PSK levels and can be restored by supplementation of exogenous PSKα peptide.

## Claims

1. An isolated plant transcription factor complex, comprising protein of the ERF transcription factors and SCL21 p, or a homologue, orthologue or paralogue thereof.

2. An isolated plant transcription factor complex according to claim 1, wherein said ERF transcription factor is ERF115p, or a homologue, orthologue or paralogue thereof.

3. The isolated plant transcription factor complex according to claim 1, wherein said complex is a heterodimeric complex.

4. The use of a transcription factor complex according to any of the claims 1 to 3 to induce phytosulphokine genes.

5. The use of a transcription factor complex according to claim 4, wherein said phytosulphokine gene is selected from the group consisting of PSK2 and PSK5.

6. The use of a transcription factor complex according to any of the claims 1 to 3 to modulate plant growth.

7. The use of a transcription factor complex according to any of the claims 1 to 3 to induce stress resistance in plant.

8. The use of a transcription factor complex according to claim 7 wherein said stress is salt stress.

9. The use of a transcription factor complex according to any of the claims 1 to 3 to induce pathogen and/or pest resistance in plant.

10. The use of a transcription factor complex according to claim 9, wherein said pathogen is a fungal pathogen.

11. The use of a transcription factor complex according to claim 9, wherein said pest is a sucking or chewing insect.

12. A recombinant plan cell, overexpressing both a gene encoding for an ERF transcription factor and SCL21, or a homologue, orthologue or paralogue thereof.

13. A transgenic plant, overexpressing both a gene encoding for an ERF transcription factor and SCL21, or a homologue, orthologue or paralogue thereof.


SEQUENCE LISTING

<110>  VIB VZW
       UNIVERSITEIT GENT

<120>  HETERODIMERIC TRANSCRIPTION FACTOR INVOLVED IN PSK SYNTHESIS

<130>  LiDV/ERFSCL/434

<160>  4

<170>  PatentIn version 3.5

<210>  1
<211>  263
<212>  PRT
<213>  Arabidopsis thaliana

<400>  1

Met Ala Asn Ser Gly Asn Tyr Gly Lys Arg Pro Phe Arg Gly Asp Glu
1               5                   10                  15

Ser Asp Glu Lys Lys Glu Ala Asp Asp Asp Glu Asn Ile Phe Pro Phe
            20                  25                  30

Phe Ser Ala Arg Ser Gln Tyr Asp Met Arg Ala Met Val Ser Ala Leu
        35                  40                  45

Thr Gln Val Ile Gly Asn Gln Ser Ser Ser His Asp Asn Asn Gln His
    50                  55                  60

Gln Pro Val Val Tyr Asn Gln Gln Asp Pro Asn Pro Pro Ala Pro Pro
65                  70                  75                  80

Thr Gln Asp Gln Gly Leu Leu Arg Lys Arg His Tyr Arg Gly Val Arg
                85                  90                  95

Gln Arg Pro Trp Gly Lys Trp Ala Ala Glu Ile Arg Asp Pro Gln Lys
            100                 105                 110

Ala Ala Arg Val Trp Leu Gly Thr Phe Glu Thr Ala Glu Ala Ala Ala
        115                 120                 125

Leu Ala Tyr Asp Asn Ala Ala Leu Lys Phe Lys Gly Ser Lys Ala Lys
    130                 135                 140

Leu Asn Phe Pro Glu Arg Ala Gln Leu Ala Ser Asn Thr Ser Thr Thr
145                 150                 155                 160

Thr Gly Pro Pro Asn Tyr Tyr Ser Ser Asn Asn Gln Ile Tyr Tyr Ser
                165                 170                 175

Asn Pro Gln Thr Asn Pro Gln Thr Ile Pro Tyr Phe Asn Gln Tyr Tyr

180                 185                 190

Tyr Asn Gln Tyr Leu His Gln Gly Gly Asn Ser Asn Asp Ala Leu Ser
        195                 200                 205

Tyr Ser Leu Ala Gly Gly Glu Thr Gly Gly Ser Met Tyr Asn His Gln
    210                 215                 220

Thr Leu Ser Thr Thr Asn Ser Ser Ser Ser Gly Gly Ser Ser Arg Gln
225                 230                 235                 240

Gln Asp Asp Glu Gln Asp Tyr Ala Arg Tyr Leu Arg Phe Gly Asp Ser
                245                 250                 255

Ser Pro Pro Asn Ser Gly Phe
            260

<210>  2
<211>  413
<212>  PRT
<213>  Arabidopsis thaliana

<400>  2

Met Asp Asn Val Arg Gly Ser Ile Met Leu Gln Pro Leu Pro Glu Ile
1               5                   10                  15

Ala Glu Ser Ile Asp Asp Ala Ile Cys His Glu Leu Ser Met Trp Pro
            20                  25                  30

Asp Asp Ala Lys Asp Leu Leu Leu Ile Val Glu Ala Ile Ser Arg Gly
        35                  40                  45

Asp Leu Lys Leu Val Leu Val Ala Cys Ala Lys Ala Val Ser Glu Asn
    50                  55                  60

Asn Leu Leu Met Ala Arg Trp Cys Met Gly Glu Leu Arg Gly Met Val
65                  70                  75                  80

Ser Ile Ser Gly Glu Pro Ile Gln Arg Leu Gly Ala Tyr Met Leu Glu
                85                  90                  95

Gly Leu Val Ala Arg Leu Ala Ala Ser Gly Ser Ser Ile Tyr Lys Ser
            100                 105                 110

Leu Gln Ser Arg Glu Pro Glu Ser Tyr Glu Phe Leu Ser Tyr Val Tyr
        115                 120                 125

Val Leu His Glu Val Cys Pro Tyr Phe Lys Phe Gly Tyr Met Ser Ala
    130                 135                 140

Asn Gly Ala Ile Ala Glu Ala Met Lys Asp Glu Glu Arg Ile His Ile
145                 150                 155                 160

Ile Asp Phe Gln Ile Gly Gln Gly Ser Gln Trp Ile Ala Leu Ile Gln
                165                 170                 175

Ala Phe Ala Ala Arg Pro Gly Gly Ala Pro Asn Ile Arg Ile Thr Gly
            180                 185                 190

Val Gly Asp Gly Ser Val Leu Val Thr Val Lys Lys Arg Leu Glu Lys
        195                 200                 205

Leu Ala Lys Lys Phe Asp Val Pro Phe Arg Phe Asn Ala Val Ser Arg
    210                 215                 220

Pro Ser Cys Glu Val Glu Val Glu Asn Leu Asp Val Arg Asp Gly Glu
225                 230                 235                 240

Ala Leu Gly Val Asn Phe Ala Tyr Met Leu His His Leu Pro Asp Glu
                245                 250                 255

Ser Val Ser Met Glu Asn His Arg Asp Arg Leu Leu Arg Met Val Lys
            260                 265                 270

Ser Leu Ser Pro Lys Val Val Thr Leu Val Glu Gln Glu Cys Asn Thr
        275                 280                 285

Asn Thr Ser Pro Phe Leu Pro Arg Phe Leu Glu Thr Leu Ser Tyr Tyr
    290                 295                 300

Thr Ala Met Phe Glu Ser Ile Asp Val Met Leu Pro Arg Asn His Lys
305                 310                 315                 320

Glu Arg Ile Asn Ile Glu Gln His Cys Met Ala Arg Asp Val Val Asn
                325                 330                 335

Ile Ile Ala Cys Glu Gly Ala Glu Arg Ile Glu Arg His Glu Leu Leu
            340                 345                 350

Gly Lys Trp Lys Ser Arg Phe Ser Met Ala Gly Phe Glu Pro Tyr Pro
        355                 360                 365

Leu Ser Ser Ile Ile Ser Ala Thr Ile Arg Ala Leu Leu Arg Asp Tyr
    370                 375                 380

Ser Asn Gly Tyr Ala Ile Glu Glu Arg Asp Gly Ala Leu Tyr Leu Gly
385                 390                 395                 400

Trp Met Asp Arg Ile Leu Val Ser Ser Cys Ala Trp Lys
                405                 410

<210>  3
<211>  87
<212>  PRT
<213>  Arabidopsis thaliana

<400>  3

Met Ala Asn Val Ser Ala Leu Leu Thr Ile Ala Leu Leu Leu Cys Ser
1               5                   10                  15

Thr Leu Met Cys Thr Ala Arg Pro Glu Pro Ala Ile Ser Ile Ser Ile
            20                  25                  30

Thr Thr Ala Ala Asp Pro Cys Asn Met Glu Lys Lys Ile Glu Gly Lys
        35                  40                  45

Leu Asp Asp Met His Met Val Asp Glu Asn Cys Gly Ala Asp Asp Glu
    50                  55                  60

Asp Cys Leu Met Arg Arg Thr Leu Val Ala His Thr Asp Tyr Ile Tyr
65                  70                  75                  80

Thr Gln Lys Lys Lys His Pro
                85

<210>  4
<211>  77
<212>  PRT
<213>  Arabidopsis thaliana

<400>  4

Met Val Lys Phe Thr Thr Phe Leu Cys Ile Ile Ala Leu Leu Leu Cys
1               5                   10                  15

Ser Thr Leu Thr His Ala Ser Ala Arg Leu Asn Pro Thr Ser Val Tyr
            20                  25                  30

Pro Glu Glu Asn Ser Phe Lys Lys Leu Glu Gln Gly Glu Val Ile Cys
        35                  40                  45

Glu Gly Val Gly Glu Glu Glu Cys Phe Leu Ile Arg Arg Thr Leu Val
    50                  55                  60

Ala His Thr Asp Tyr Ile Tyr Thr Gln Asn His Asn Pro
65                  70                  75